# EUROPEAN PATENT SPECIFICATION

(11) **EP 1 531 788 B1**
(45) Date of publication and mention of the grant of the patent: **31.01.2007**
(21) Application number: 03784006.3
(22) Date of filing: 01.07.2003
(51) Int. Cl.: A61K 8/26, A61K 8/92, A61Q 15/00

(54) **ANTIPERSPIRANT AEROSOL COMPOSITIONS**
SCHWEISSHEMMENDE AEROSOLPRÄPARATE
COMPOSITIONS AEROSOL ANTISUDORIFIQUES

(30) Priority: 24.07.2002 GB 0217256
(43) Date of publication of application: 25.05.2005
(73) Proprietor: UNILEVER PLC, London EC4P 4BQ (GB); UNILEVER N.V., 3013 AL Rotterdam (NL)
(72) Inventor: BROWN, Nathan Charles, Unilever R&D Port Sunlight, Wirral, Merseyside CH63 3JW (GB); RIELEY, Hugh, Unilever R&D Port Sunlight, Wirral, Merseyside CH63 3JW (GB); SHEARMUR, Thomas,E. Unilever R&D Port Sunlight, Wirral, MerseysideCH63 3JW (GB)
(74) Representative: Whaley, Christopher
(86) International application number: PCT/EP2003/007065
(87) International publication number: WO 2004/014330

(56) References cited:
- EP-A- 0 804 921
- WO-A-97/47273
- WO-A-98/35648
- WO-A-02/055044
- US-A- 5 840 289
- US-A1- 2002 037 264
- US-B1- 6 245 324
- US-B1- 6 251 376
- [Online] retrieved from WWW.EXXONMOBILCHEMICAL.COM/PUBLIC_FILES/SY NTHETICS/SYNTHETIC_LUBRICANTS_AND_FLUIDS/W ORLDWIDE/PURESYN

## Description

This invention relates to antiperspirant aerosol compositions suitable for topical application to the human body and to methods of making the same.

Antiperspirant compositions suitable for topical application typically comprise an antiperspirant material such as aluminium chlorohydrate which acts to suppress the level of perspiration on the area of the body to which it is applied. Antiperspirant compositions are widely applied in the form of a pressurised, propellant driven aerosol spray.

Aerosol antiperspirant compositions typically have the antiperspirant material suspended in an anhydrous carrier fluid, together with a propellant and a suspending agent. Other minor ingredients can also be present, and the composition is housed in a pressurised container.

A major problem associated with all antiperspirants, including aerosol antiperspirants, is that of deposition or whitening where the antiperspirant active, which is usually in powder form, is deposited on a user's clothes or skin, thereby causing visible whitening/staining and occasionally damage to the clothes.

Various attempts have been made to reduce the whitening or visible deposits. A widely used approach has been to incorporate a masking oil such as an aliphatic hydrocarbon, ester oil, or silicone fluid into the formulation.

The masking oil functions by coating the surface of the antiperspirant active particles and minimises the scattering of light, thereby rendering the active less visible to the naked eye.

The masking method has been found to reduce whitening in stick and roll-on products. The reduction of whitening caused by aerosol compositions containing activated aluminium chlorohydrate (AACH) as the active is more difficult. This is in part due to the difficulty in matching the refractive index (RI) of the masking oil with that of AACH, as commercially available AACH does not have a continuous RI value throughout the particle. More particularly, AACH particles generally contain hollow cores, having an RI of 1.0 while the RI of the outer particle is in the region of 1.5 which results in visible whitening.

One approach to reducing the whitening problem with AACH aerosol compositions is described in US 5,840,289 (Hall), wherein the AACH is milled to reduce the number of particles having hollow cores and the resulting active is masked with a masking oil of appropriate RI.

The inventors have now discovered that good antiperspirancy and remarkably low whitening can be achieved with AACH aerosol compositions comprising milled AACH having non-hollow particles in combination with a masking oil of particularly high viscosity. The good antiperspirancy found with compositions of the invention may enable a lower amount of antiperspirant active to be used whilst still maintaining the antiperspirancy efficacy of a conventional aerosol antiperspirant composition having a higher level of antiperspirant active.

A further benefit that may be achieved by use of the present invention is the enhanced maintenance of the antiperspirant active on the skin. This enables reduced transfer of the antiperspirant active to clothes worn in close proximity to the skin, thereby reducing whitening of said clothes and helps to maintain the antiperspirant benefit sought by the consumer.

Thus, according to a first aspect of the invention, there is provided a suspension antiperspirant aerosol composition comprising milled activated aluminium chlorohydrate having non-hollow particles and a carrier fluid comprising a masking oil of kinematic viscosity at 25°C of 10³ mm²/s or greater.

According to a second aspect of the invention, there is provided a method of manufacture of a suspension antiperspirant aerosol composition, said method comprising the suspension of a milled activated aluminium chlorohydrate (AACH) having non-hollow particles in a carrier fluid comprising a masking oil of kinematic viscosity at 25°C of 10³ mm²/s or greater.

According to a third aspect of the invention, there is provided a method of reducing perspiration and giving low visible deposits comprising the application to the human body of a suspension antiperspirant aerosol composition comprising milled AACH having non-hollow particles and a carrier fluid comprising a masking oil of kinematic viscosity at 25°C of 10³ mm²/s or greater.

Without wishing to be bound by theory, the applicant believes that the reduced white deposits obtained with antiperspirant compositions of the present invention is due to the particular morphology of the antiperspirant actives selected and their highly effective coating by the particularly high viscosity masking oils indicated.

The chemical nature and preparation of AACH antiperspirant actives suitable for use in the present invention are described in GB 1,568,831, GB 1,597,497, GB 1,597,498, EP 6,738, EP 6,739, EP 7,191, EP 191,628, EP 256,832 and EP 491,395, the contents of which are incorporated herein by reference. The amount of AACH present in the compositions of the invention may be from 1 to 30% and particularly from 2 to 20%, the upper level of AACH preferably being 10% or less and especially being 5% or less, all percentages being by weight of the composition.

The AACH used in the present invention is milled and has non-hollow particles. Preferably, a high level of non-hollow AACH particulate material is present, the hollows in the particles comprising less than 40% of the total volume of the AACH. It is particularly preferred that the hollows comprise less than 20%, and especially less than 10%, of the total volume of the AACH. AACH in which hollows comprise less than 5% of the total volume of the active is most preferred. Measurement of the amount of non-hollow AACH particulate material may be performed by a suitable microscopy technique (e.g. SEM) in combination with suitable image analysis.

Suitably, the AACH used in the present invention has a continuous RI. The RI of the AACH is preferably between 1.52 to 1.57. The mean particle size of the AACH is preferably from 20 to 30 microns (measured as the D[4,3], using a light scattering technique). Typically the AACH is prepared by milling a sample having a mean particle size (measured as above) of from 50 to 150 micron and comprising hollow particles.

The masking oil used in the present invention has a particularly high viscosity, being of viscosity 10³ nm²/s or greater. The viscosity values quoted in this specification are kinematic viscosities, measured at 25°C. A suitable method for measuring kinematic viscosities utilises a glass capillary viscometer, for example, as described in Dow Corning's Corporate Test Method CTM 0004, of July 29, 1970.

The masking oil may be any suitable oil of appropriate viscosity, examples including hydrocarbon oils, ester oils, and silicone oils. Suitable hydrocarbon oils include hydrogenated polyolefins, in particular hydrogenated polydecenes, examples including PureSyn oils of viscosity 10³ mm²/s or greater, such as PureSyn 1000 or 3000, available from Exxon-Mobil. Suitable silicone oils include linear and crosslinked polydimethylsiloxane (PDMS) fluids, examples including the DC200 PDMS fluids of viscosity 10³ mm²/s or greater, available from Dow Corning.

The RI of the masking oil is preferably from 1.40 to 1.57. Silicone oils are particularly preferred. The viscosity of the masking is 10³ mm²/s or greater, preferably 10⁴ mm²/s or greater, and more preferably 30,000 mm²/s or greater.

The masking oil may be used in an amount from 0.5% or 1%, up to 30%, in particular up to 10%, and especially up to 5%, all percentages being by weight of the total composition.

The masking oil may function as an emollient oil and/or lubricant. It may also serve to facilitate uniform distribution of the antiperspirant material on the skin.

The carrier fluid is any oil suitable for suspending ACH and compatible with the masking oil comprising it. Typically, the carrier fluid is present at a level of from 1 to 30%, in particular from 2 to 20%, and especially from 5 to 10% by weight of the composition.

The carrier fluid may comprise an emollient oil, additional to any masking oil also having this function (*vide supra*). Suitable emollient oils are disclosed in US Patent Nos. 4,822,596 and 4,904,463, the disclosures of which are incorporated by reference herein. Preferred emollient oils are alkyl esters, such as PurSyn Ester 2E7 (neopentylglycol dihexanoate), ex Exxon-Mobil; benzoate esters such as Finsolv TN (Trade Mark), ex Finetex Inc.; hydrogenated polyalkenes, such as Panalane, ex Amoco and PureSyn PAO 2 (hydrogenated polydecene), ex Exxon-Mobil; PPG ethers, such as Fluid AP (PPG-14 butylether), ex Union Carbide; isopropyl palmitate; phenylsilicone; and isopropyl myristate.

It is particularly preferred that the carrier fluid comprises a volatile silicone fluid. Preferred volatile fluids include dimethyl cyclosiloxanes, such Dow Corning fluids DC244, DC245, DC344 and DC345. The combination of such materials and the masking oil may be used at a level of from 1 to 30%, in particular from 2 to 20%, and especially from 5 to 10% by weight of the composition.

In order to prevent caking or settling out of the antiperspirant salt from the carrier fluid, a bulking or suspending agent is preferably incorporated into the composition of the invention. The suspending agent is preferably hydrophobically treated montmorillionite clay such as a bentonite or a hectorite. One such commercially available clay is Bentone 38V, which is a hectorite clay available from NL Industries, Inc. The amount of clay in the composition of the invention may be from 0.2 to 5.0% by weight of the total composition.

A propellant gas is generally present as part of the compositions of the invention. Any liquefiable gas known in the art for use in propellant driven aerosol products may be used. Examples of suitable propellants include trichlorofluoromethane, trichlorotrifluoromethane, difluoroethane, propane, butane or isobutane or combinations thereof. When used, the amount of liquefied gas in the composition of the invention is typically from 5 to 95% and preferably from 30 to 90% by weight of the composition. Alternatively, compressed gas such as air, nitrogen, or carbon dioxide may be used as propellant.

Other minor ingredients which may be present in the compositions of the invention include:
- cosmetically acceptable carrier fluid components, such as straight and branched chain alcohols, for example, ethanol, isobutanol or isopropanol;
- deodorant active perfumes and deodorant compounds which can act as antimicrobial agents;
- hydrophobic oils, such as liquid paraffin oils;
- inorganic electrolytes, such as sodium chloride or sodium sulphate;
- other thickeners such as clays, silicas, for example, Aerosil 200 and hydroxypropyl celluloses such as Klucel;
- polar additives such as propylene carbonate or alcohol;
- skin feel improvers, such as talc and finely divided polyethylene such as Accumist B18;
- humectants, such as polyols, for example glycerol;
- perfumes;
- preservatives and antioxidants;
- skin benefit agents such as allantoin;
- colours;
- other cosmetic adjuncts conventionally employed in propellant driven aerosol products.

The compositions of the invention may be manufactured by a method comprising the suspension of a milled activated aluminium chlorohydrate (AACH) having non-hollow particles in a carrier fluid comprising a masking oil of viscosity 10³ mm²/s or greater. Techniques employed in the manufacture of conventional suspension AP aerosol products may be employed. In a particular method of manufacture, the AACH is suspended in a pre-formed mixture comprising volatile silicone, masking oil, and perfume, and said suspension is then placed in an aerosol can, preferably made of aluminium or tinplate, and a propellant gas is added in a liquefied form.

### Examples

The compositions indicated in the Tables below were prepared in the following manner. The volatile silicone, other oil (silicone oil or hydrocarbon oil), and Bentone 38V where sheared together at 9000 rpm for 5 minutes using a Silverson L4RT mixer. The perfume was then added whilst shearing and shearing was continued for a further 2 minutes. Shearing was then discontinued and the AACH was dispersed in the mixture using a spatula. The mixture was then subjected to a further 5 minutes shearing using the Silverson L4RT mixer.

The whiteness scores were obtained using the following method. The fully formulated compositions were each sprayed directly onto black test cloths from a distance of 25 cm, using a standard spray nozzle and a duration of 5 seconds per cloth. After approximately one hour, the whiteness scores of the cloths were measured using an image analysis technique. The results were analysed using standard statistical techniques to give the average whiteness score from each composition and the 95% confidence limits.

Comparative examples are indicated by letter codes.

Compositional amounts are given as percentages by weight.

**Table 1: Compositions comprising 3% masking oil**

| | **Examples** | | | |
|---|---|---|---|---|
| | **A** | **B** | **C** | **1** |
| **Components** | | | | |
| A296¹ | 2 | 2 | - | - |
| Alloxicol LR² | - | - | 2 | 2 |
| Bentone 38V³ | 0.5 | 0.5 | 0.5 | 0.5 |
| Volatile silicone⁴ | 6.9 | 6.9 | 6.9 | 6.9 |
| DC 200 (50)⁵ | 3 | - | 3 | - |
| DC 200 (30,000)⁶ | - | 3 | - | 3 |
| Fragrance | 0.6 | 0.6 | 0.6 | 0.6 |
| CAP 40⁷ | To 100 | To 100 | To 100 | To 100 |

| **Whiteness** | | | | |
|---|---|---|---|---|
| Score: | **1789** | **1397** | **1420** | **750** |
| 95% limits: | ±145 | ±89 | ±64 | ±57 |

| | | | | |
|---|---|---|---|---|
| 1. Conventional unmilled AACH, ex BK Giulini. 2. Milled AACH, ex BK Giulini. 3. Suspending agent, hectorite clay, ex NL Industries. 4. DC245, ex Dow Corning. 5. PDMS of viscosity 50 mm²/s, ex Dow Corning. 6. PDMS of viscosity 30,000 mm²/s, ex Dow Corning. 7. Propellant, proprietary mix of butane, isobutane and propane, ex. Calor. | | | | |

These results illustrate that use of a milled active having non-hollow particles (Alloxicol LR) and a masking oil having a particularly high viscosity (DC 200 (30,000 mm²/s) leads to a particularly low whiteness score (Example 1). Poorer (i.e. higher) whiteness scores where obtained when the composition comprised only one of the required features (Examples B and C) and an even poorer whiteness score was obtained when the composition comprised neither of the required features (Example A).

**Table 2: Compositions comprising 1% masking oil**

| | **Examples** | | | |
|---|---|---|---|---|
| | **D** | **2** | **3** | **4** |
| **Components** | | | | |
| Alloxicol LR¹ | 2 | 2 | 2 | 2 |
| Bentone 38V¹ | 0.5 | 0.5 | 0.5 | 0.5 |
| Volatile silicone¹ | 8.9² | 8.9 | 8.9 | 8.9 |
| DC 200 (50)¹ | 1 | - | - | - |
| DC 200 (1000)³ | - | 1 | - | - |
| DC 200 (30,000)¹ | - | - | 1 | - |
| Puresyn 1000⁴ | - | - | | 1 |
| Fragrance | 0.6 | 0.6 | 0.6 | 0.6 |
| CAP 40¹ | To 100 | To 100 | To 100 | To 100 |

| **Whiteness** | | | | |
|---|---|---|---|---|
| Score: | **1429** | **1213** | **848** | **1064** |
| 95% limits: | ±123 | ±210 | ±35 | ±8 |

| | | | | |
|---|---|---|---|---|
| 1. As defined in Table 1. 2. Including a small amount of silicone gum (Q2-1501, ex Dow Corning). 3. PDMS of viscosity 1,000 mm²/s, ex Dow Corning. 4. Hydrogenated polydecene of viscosity 42,300 mm²/s, ex Exxon-Mobil. | | | | |

These results illustrate the benefit of using a masking oil of viscosity 10³ mm²/s or greater, whether the masking oil be a silicone oil or a hydrocarbon oil.

The results from silicone oil containing Examples D, 2, and 3 are shown again in Figure 1, which suggests that a logarithmic-linear relationship between viscosity and whiteness score may be present.

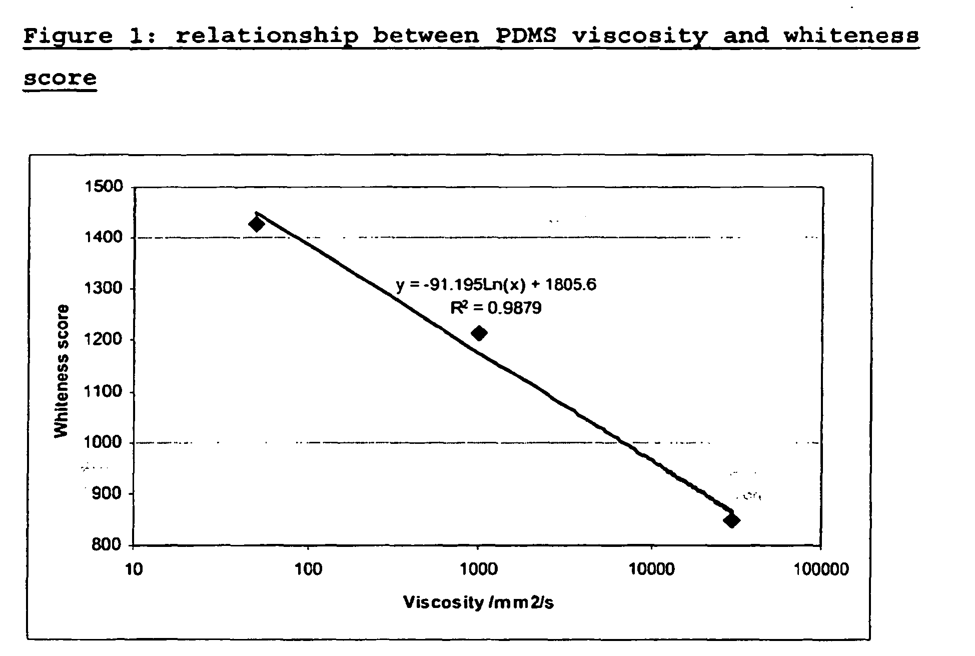

Further Examples of compositions according to the invention are illustrated in Table 3.

**Table 3**

| | **Examples** | | | |
|---|---|---|---|---|
| | **5** | **6** | **7** | **8** |
| **Components** | | | | |
| Alloxicol LR¹ | 2 | 2 | 2 | 2 |
| Bentone 38V¹ | 0.5 | 0.5 | 0.5 | 0.5 |
| Volatile silicone¹ | 9 | - | - | - |
| Finsolv TN² | - | 8 | - | 8 |
| PureSyn Ester 2E7³ | - | - | 9 | - |
| DC 200 (30,000)¹ | - | - | 1 | 0.5 |
| PureSyn 3000⁴ | 1 | 1 | - | 0.5 |
| Fragrance | 0.6 | 0.6 | 0.6 | 0.6 |
| CAP 40¹ | To 100 | To 100 | To 100 | To 100 |

| | | | | |
|---|---|---|---|---|
| 1 As defined in Table 1. 2 C₁₂₋₁₅ alkyl benzoate, ex Finetex Inc. 3 Neopentylglycol dihexanoate, ex Exxon-Mobil. 4 Hydrogenated polydecene of viscosity 60,000 mm²/s, ex Exxon-Mobil. | | | | |

## Claims

1. A suspension antiperspirant aerosol composition comprising milled activated aluminium chlorohydrate (AACH) having non-hollow particles and a carrier fluid comprising a masking oil of kinematic viscosity at 25°C of 10³ mm²/s or greater.

2. An aerosol composition according to claim 1, wherein the AACH is present at a level of from 1% to 30% by weight of the composition.

3. An aerosol composition according to claim 2 wherein the AACH is present at a level of from 2% to 5% by weight of the composition.

4. An aerosol composition according to any of the preceding claims, wherein the masking oil has a kinematic viscosity at 25°C of 30,000 mm²/s or greater.

5. An aerosol composition according to any of the preceding claims, wherein the AACH has a continuous RI.

6. An aerosol composition according to any of the preceding claims, wherein the masking oil has an RI of 1.40 to 1.57.

7. An aerosol composition according to claim 6, wherein the masking oil is a silicone oil.

8. An aerosol composition according to any of the preceding claims, comprising an additional emollient oil.

9. An aerosol composition according to any of the preceding claims, comprising a bulking or suspending agent.

10. An aerosol composition according to any of the preceding claims, comprising a volatile silicone.

11. An aerosol composition according to any of the preceding claims, comprising a propellant gas.

12. An aerosol composition according to claim 11, wherein the propellant gas is a liquefied gas at a level of from 5 to 95% by weight of the composition.

13. A method of manufacture of a suspension antiperspirant aerosol composition, said method comprising the suspension of a milled activated aluminium chlorohydrate (AACH) having non-hollow particles in a carrier fluid comprising a masking oil of kinematic viscosity at 25°C of 10³ mm²/s or greater.

14. A non-therapeutic method of reducing perspiration and giving low visible deposits comprising the application to the human body of a suspension antiperspirant aerosol composition comprising milled AACH having non-hollow particles and a carrier fluid comprising a masking oil of kinematic viscosity at 25°C of 10³ mm²/s or greater.

## Patentansprüche

1. Suspendierte schweißhemmende Aerosolzusammensetzung, umfassend gemahlenes aktiviertes Aluminiumchlorhydrat (AACH), das nicht-hohle Teilchen aufweist, und ein Trägerfluid, umfassend ein Maskierungsöl mit einer kinematischen Viskosität bei 25 °C von 10³ mm²/s oder mehr.

2. Aerosolzusammensetzung nach Anspruch 1, wobei das AACH bei einem Gehalt von 1 bis 30 Gew.-% der Zusammensetzung vorliegt.

3. Aerosolzusammensetzung nach Anspruch 2, wobei das AACH bei einem Gehalt von 2 bis 5 Gew.-% der Zusammensetzung vorliegt.

4. Aerosolzusammensetzung nach einem der vorhergehenden Ansprüche, wobei das Maskierungsöl eine kinematische Viskosität bei 25 °C von 30.000 mm²/s oder mehr hat.

5. Aerosolzusammensetzung nach einem der vorhergehenden Ansprüche, wobei das AACH einen konstanten RI hat.

6. Aerosolzusammensetzung nach einem der vorhergehenden Ansprüche, wobei das Maskierungsöl einen RI von 1,40 bis 1,57 hat.

7. Aerosolzusammensetzung nach Anspruch 6, wobei das Maskierungsöl Silikonöl ist.

8. Aerosolzusammensetzung nach einem der vorhergehenden Ansprüche, umfassend ein zusätzliches Erweichungsöl.

9. Aerosolzusammensetzung nach einem der vorhergehenden Ansprüche, umfassend ein Füll- oder Suspendiermittel.

10. Aerosolzusammensetzung nach einem der vorhergehenden Ansprüche, umfassend ein flüchtiges Silikon.

11. Aerosolzusammensetzung nach einem der vorhergehenden Ansprüche, umfassend ein Treibgas.

12. Aerosolzusammensetzung nach Anspruch 11, wobei das Treibgas ein Flüssiggas mit einem Gehalt von 5 bis 95 Gew.-% der Zusammensetzung ist.

13. Verfahren zur Herstellung einer suspendierten schweißhemmenden Aerosolzusammensetzung, wobei das Verfahren die Suspension von gemahlenem aktiviertem Aluminiumchlorhydrat (AACH), das nicht-hohle Teilchen aufweist, in einem Trägerfluid, umfassend ein Maskierungsöl mit einer kinematischen Viskosität bei 25 °C von 10³ mm²/s oder mehr, umfaßt.

14. Nicht therapeutisches Verfahren zur Verringerung des Schwitzens und zur Abgabe von weniger sichtbaren Abscheidungen, umfassend das Auftragen einer suspendierten schweißhemmenden Aerosolzusammensetzung, umfassend gemahlenes aktiviertes Aluminiumchlorhydrat (AACH), das nicht-hohle Teilchen aufweist, und ein Trägerfluid, umfassend ein Maskierungsöl mit einer kinematischen Viskosität bei 25 °C von 10³ mm²/s oder mehr, auf den menschlichen Körper.

## Revendications

1. Composition anti-transpirante en aérosol en suspension comprenant du chlorhydrate d'aluminium activé (AACH) broyé dont les particules sont non creuses et un liquide porteur comprenant une huile masquante dont la viscosité cinématique est supérieure ou égale à 10³ mm²/s à 25°C.

2. Composition en aérosol selon la revendication 1, dans laquelle l'AACH est présent en une proportion valant de 1% à 30% en poids de la composition.

3. Composition en aérosol selon la revendication 2, dans laquelle l'AACH est présent en une proportion valant de 2% à 5% en poids de la composition.

4. Composition en aérosol selon l'une quelconque des revendications précédentes, dans laquelle l'huile masquante présente une viscosité cinématique supérieure ou égale à 30000 mm²/s à 25°C.

5. Composition en aérosol selon l'une quelconque des revendications précédentes, dans laquelle l'AACH présente un indice de réfraction RI continu.

6. Composition en aérosol selon l'une quelconque des revendications précédentes, dans laquelle l'huile masquante présente un indice de réfraction RI valant de 1,40 à 1,57.

7. Composition en aérosol selon la revendication 6, dans laquelle l'huile masquante représente une huile de silicone.

8. Composition en aérosol selon l'une quelconque des revendications précédentes, comprenant une huile émolliente supplémentaire.

9. Composition en aérosol selon l'une quelconque des revendications précédentes, comprenant un agent de suspension ou un diluant.

10. Composition en aérosol selon l'une quelconque des revendications précédentes, comprenant un composé de silicone volatil.

11. Composition en aérosol selon l'une quelconque des revendications précédentes, comprenant un gaz propulseur.

12. Composition en aérosol selon la revendication 11, dans laquelle le gaz propulseur représente un gaz liquéfié en une proportion valant de 5 à 95% en poids de la composition.

13. Procédé de fabrication d'une composition anti-transpirante en aérosol en suspension, ledit procédé comprenant la mise en suspension du chlorhydrate d'aluminium activé (AACH) broyé dont les particules sont non creuses dans un liquide porteur comprenant une huile masquante dont la viscosité cinématique est supérieure ou égale à 10³ mm²/s à 25°C.

14. Procédé non thérapeutique permettant de réduire la transpiration et de générer peu de dépôts visibles qui comprend le fait d'appliquer sur le corps humain une composition anti-transpirante en aérosol en suspension comprenant de l'AACH broyé dont les particules sont non creuses et un liquide porteur comprenant une huile masquante dont la viscosité cinématique est supérieure ou égale à 10³ mm²/s à 25°C.
